# EUROPEAN PATENT APPLICATION

(11) **EP 1 923 093 A1**
(43) Date of publication of application: **21.05.2008**
(21) Application number: 07120920.9
(22) Date of filing: 16.11.2007
(51) Int. Cl.: A61N 1/08, A61N 1/32, A61N 1/04

(54) **Low-frequency electric therapy apparatus**

(30) Priority: 20.11.2006 JP 2006312473
(71) Applicant: Omron Healthcare Co., Ltd., Ukyo-ku Kyoto-shi Kyoto 615-0084 (JP)
(72) Inventor: Miki, Akitoshi, Kyoto 615-0084 (JP); Mizuta, Yoshikazu, Kyoto 615-0084 (JP); Asai, Rika, Kyoto 615-0084 (JP)
(74) Representative: Wilhelms . Kilian & Partner Patentanwälte

(57) **Abstract**

A low-frequency electric therapy apparatus is provided with: a pair of pads 300 and 300 each of which can change the position and the area of an energizing area; and a wave form generation and output portion 250 which applies a treating current between an energizing area of one pad 300 and that of the other pad 300. While the wave form generation and output portion 250 outputs the treating current, the output portion 250 produces a kneading stimulus or a rubbing stimulus by changing the position and/or the area of the energizing area of at least one pad. Thereby, there is provided a low frequency electric therapy apparatus having a simple configuration which may produce more similar bodily sensation to that of "kneading", and "rubbing" performed by a person, and has excellent convenience.

## Description

### BACKGROUND OF THE INVENTION

### Field of the Invention

The present invention relates to a low-frequency electric therapy apparatus.

### Description of the Related Art

There has been known a low-frequency electric therapy apparatus, by which a treating current of a low frequency is energized between electrode pads applied to a body for treatment. A conventional low-frequency electric therapy apparatus has produced a stimulus corresponding to various kinds of massages such as "kneading", "tapping", "rubbing", and "pushing" by devising a wave form of a treating current (refer to Japanese Patent Application Laid-Open No. 2000-14802).

However, there has been a great difference between bodily sensation of massages performed by a person and that of the low-frequency electric therapy apparatus with regard to "kneading" and "rubbing". The reason is that, while "kneading" and "rubbing" performed by a person have produced massaging effects by changing an angle for applying force, or by moving a hand touching place, it is difficult in the case of the low-frequency electric therapy apparatus to reproduce the above-described movement of a stimulus because the pad is applied (fixed) on the body.

On the other hand, there has been proposed a method for moving a stimulus portion by a configuration in which a lot of electrodes are provided on a single pad, and a combination of electrodes for forming a current path are sequentially switched (refer to Japanese Patent No. 2917270 (Japanese Patent Application Laid-Open No. 09-38215)). Moreover, there has been proposed a method in which four couples (8 pieces) of pads (electrodes) are applied on a body at predetermined intervals, and a stimulus portion is moved by sequentially switching a pad couple for output an current (refer to Japanese Patent Application Laid-Open No. 2003-245363).

By the way, when a person massages a portion such as shoulders and a waist, it is usual to perform treating on both right side and left side, using both hands. In order to obtain more similar bodily sensation by the low-frequency electric therapy apparatus to that of massaging by a person, it is also considered important to produce similar bodily sensation to that of massaging with the both hands.

Considering the above point, a single pad configuration described in JP-A No. 09-38215 has a disadvantage that treatment may not be given at the same time to portions such as both shoulders and the both sides of the waist, which are remote from each other, because a pad is applied only one portion of a body (for example, only a right shoulder). However, a configuration, in which a lot of pad couples are provided as described in JP-A No. 2003-245363 is not preferable because a complicated circuit configuration and an increased number of components, accordingly, an increased cost are caused. Moreover, in a case of an increased number of pads, there is caused another use problem that it is difficult and troublesome to apply the increased number of pads at their appropriate positions under a preferable combination of the pads.

### SUMMARY OF THE INVENTION

The present invention has been made, considering the above described circumstances, and its object is to provide a low-frequency electric therapy apparatus having a simple configuration which may produce more similar bodily sensation to that of "kneading", and "rubbing" caused by a person, and has excellent convenience.

In order to achieve the above-described object, a low-frequency electric therapy apparatus according to the present invention adopts the following configuration.

The low-frequency electric therapy apparatus according to the present invention is provided with: a pair of pads each of which is capable of changing the position and the area of an energizing area; and an output portion by which a treating current flows between the energizing area of one pad and that of the other pad, wherein the above-described output portion produces kneading stimulus or rubbing stimulus by changing the position and/or the area of the energizing area of at least one pad while outputting the treating current. Here, "energizing area" means the whole area, or a part of the whole area of the pad, and a range within which the treating current flows.

According to the above configuration, bodily sensation that a stimulus position or a force applying angle changes may be produced by changing the position and the area of the energizing area, and thus, a user may have a bodily sensation very similar to that of "kneading", or "rubbing" performed by a person. Moreover, as there are two pads, treatment may be given at the same time for portions apart from each other such as the right and the left shoulders, the right and the left sides of the waist or the back, the right and the left arms, and the right and left legs. Moreover, as the position and the area of the energizing area on each of the two pads may be separately changed, the bodily sensation that "kneading" and "rubbing" are given to each of the portions by the both hands may be produced. Moreover, the attachment of pads is simple because only two pads are required to be applied. Furthermore, there is another advantage that only a simple and low-cost configuration is required for the above configuration, because there is required, as a current path, only one system between the energizing area of one pad and that of the other pad.

In the case of the kneading stimulus, the above-described output portion, preferably, changes the energizing area at a speed of once in 0.5 seconds through 5 seconds. When the energizing area is changed at such a comparatively slow speed, there is obtained a bodily sensation very similar to that of "kneading" performed by a person.

On the other hand, in the case of the rubbing stimulus, it is preferable that the above-described output portion changes the energizing area at a speed of once in 0.1 seconds through 0.5 seconds. When the energizing area is changed at such a comparatively fast speed, there is obtained a bodily sensation very similar to that of "rubbing" performed by a person.

Preferably, the above-described pad has a plurality of electrodes which may separately be switched between the conducting and the non-conducting states, and the position and the area of the energizing area are changed by changing the energized electrode. The change of the position and the area of the energizing area may be realized in a simple manner by the above configuration.

When the above-described pad has an electrode on one end portion and an electrode in the center portion, it is preferable that the above-described output portion produces the kneading stimulus by alternately making the above-described electrode on one end portion and the above-described electrode in the center portion conductive. Alternatively, it is preferable that the above-described output portion produces the kneading stimulus by alternately making all of the electrode and a part of the electrodes on the pad conductive. A bodily sensation very similar to that of "kneading" performed by a person may be produced by the above-described control.

It is preferable that the above-described output portion produces the rubbing stimulus by moving the conducting electrode one by one in the pad. By the above control, there may be produced a bodily sensation very similar to that of "rubbing" performed by a person.

Preferably, the above-described output portion outputs symmetrical kneading stimulus or rubbing stimulus from the both pads by making the change patterns of the energizing areas for the two pads in agreement with each other, or by mutually reversing the change patterns. Bodily sensation that simultaneous massaging by the both hands is given may be realized by the above control.

Acceptably, the above-described output portion fixes the area of the energizing area of one pad at a maximum value, and the kneading stimulus or the rubbing stimulus is configured to be output from the other pad. As there is a characteristic that the smaller area of the energizing area causes the stimulus to become stronger, a weaker static stimulus is output from a pad having the fixed area, and a dynamic kneading stimulus or a rubbing stimulus is output from the other pad. Thereby, a user may have a bodily sensation that a person applies the palm of one hand onto a treatment portion, and kneading or rubbing is given to the person by the other hand. At this time, it is recommendable for the above-described output portion to periodically alternate the pad having the fixed area of the energizing area and the pad outputting the kneading stimulus or the rubbing stimulus. Thereby, a user may have a bodily sensation that massaging is alternately performed.

It is preferable for the above-described output portion to switch between a first mode and a second mode, wherein the first mode produces the kneading stimulus or the rubbing stimulus by changing the position and/or the area of the energizing area for at least one pad while outputting the treating current, and the second mode produces the kneading stimulus or the rubbing stimulus by changing the wave form of the treating current under a state that the position and the area of the energizing area are fixed. Thereby, the convenience is improved because a user may adequately use two modes (stimulus) to suit the preferences of the user.

It is preferable that the apparatus further provided with a display portion displaying energizing areas in each of the pads. By seeing the above display, a user may easily confirm what type of massaging is performed.

The present invention may be understood as a low-frequency electric therapy apparatus having at least a portion of the above-described means, as a method for generating a stimulus in a low-frequency electric therapy apparatus including at least a part of the above-described processing, as a program realizing the above method, or as a recording medium recording the above program. Here, each of the above-described means and the above-described processing may be combined each other to form the present invention.

According to the present invention, there may be provided a low-frequency electric therapy apparatus having a simple configuration which may produce more similar bodily sensation to that of "kneading" and "rubbing" performed by a person, and has excellent convenience.

### BRIEF DESCRIPTION OF THE DRAWINGS

FIG. 1 is a drawing showing an external view of the low-frequency electric therapy apparatus;
FIG. 2 is a drawing showing an external view of an operation portion;
FIG. 3 is a block diagram schematically showing a configuration of the low-frequency electric therapy apparatus;
FIG. 4 is a drawing showing one example of treatment wave forms using pulse currents;
FIG. 5A through FIG. 5F are a drawing showing a display example of a display portion caused by change in the energizing area;
FIG. 6 is a drawing showing one example of energizing patterns of "kneading";
FIG. 7 is a drawing showing one example of the energizing patterns of "kneading";
FIG. 8 is a drawing showing one example of the energizing patterns of "kneading"; and
FIG. 9 is a drawing showing one example of the energizing patterns of "rubbing".

### DESCRIPTION OF THE EMBODIMENTS

Hereinafter, preferable embodiments according to the present invention will be illustratively explained in detail, referring to the drawings.

### <External View of Low-frequency electric therapy apparatus >

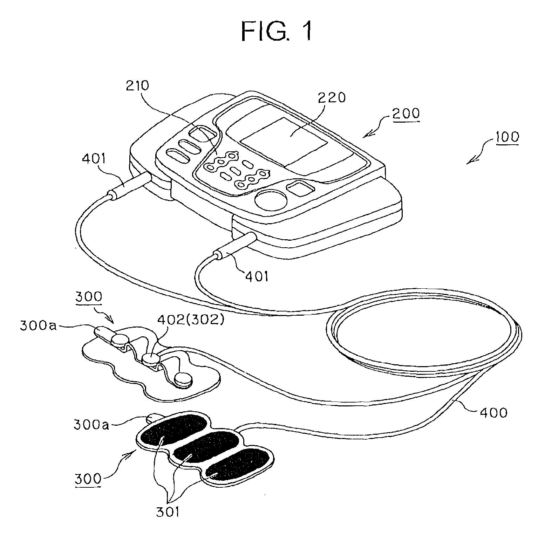

A low-frequency electric therapy apparatus according to an embodiment of the present invention will be explained, referring to FIG. 1. FIG. 1 is a drawing showing an external view of the low-frequency electric therapy apparatus.

A low-frequency electric therapy apparatus 100 roughly includes: a main body 200 of the therapy apparatus; a pair of pads 300 for being applied to a treatment portion; and a cord 400 electrically connecting a main body 200 of the therapy apparatus and the pads 300.

The pad 300 is made of a thin and flexible material. A plurality of electrodes 301 are formed on one surface (a surface contacted with the body) of the pad 300. In the present embodiment, three electrodes 301 are arranged in the longitudinal direction of the pad 300. A convex holding portion 300a is provided on one end portion in the longitudinal direction of the pad 300. This holding portion 300a functions as a mark for determining which side of the pad 300 is the upper side.

The surface of the electrode side of the pad 300 is coated with an adhesive material in a gel state. The film of the gel material is uniformly formed over the whole surface of the pad. The electrodes are not especially electrically insulated from each other, because of film of the gel material therebetween.

A snap 302 corresponding to each of the electrodes 301 is provided on the other surface of the pad 300. The main body 200 and the pad 300 (electrodes 301) are connected to each other by snap connection between a snap 402 of the code 400 and the snap 302 on the side of the pad 300 and by inserting a plug 401 of the cord 400 into a jack in the main body 200.

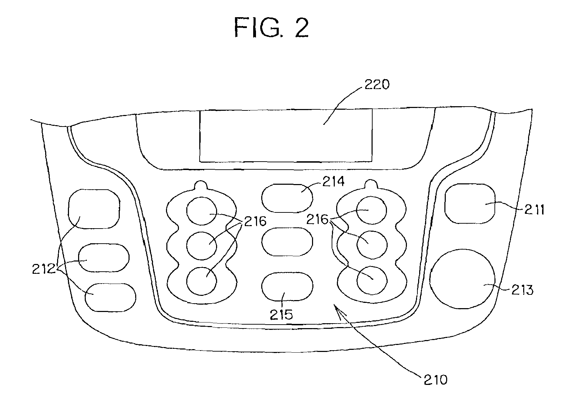

An operation portion 210 including switches and dials, and a display portion 220 including a liquid crystal display (LCD) are provided on the main body 200. As shown in FIG. 2, there are provided on the operation portion 210: a power supply switch 211 for switching on and off of the power supply; a mode selection switch 212 for selecting a treatment mode; an intensity adjustment dial 213 for adjusting the intensity; a position change switch 214 for moving an energizing area; a size change switch 215 for changing the size (area) of the energizing area; an electrode switch 216 for selecting an electrode 301 in an individual manner. A current operation status, a navigating state for operations and pad applying, and messages are displayed in the LCD on the display portion 220.

### <Internal Configuration of Low-frequency electric therapy apparatus>

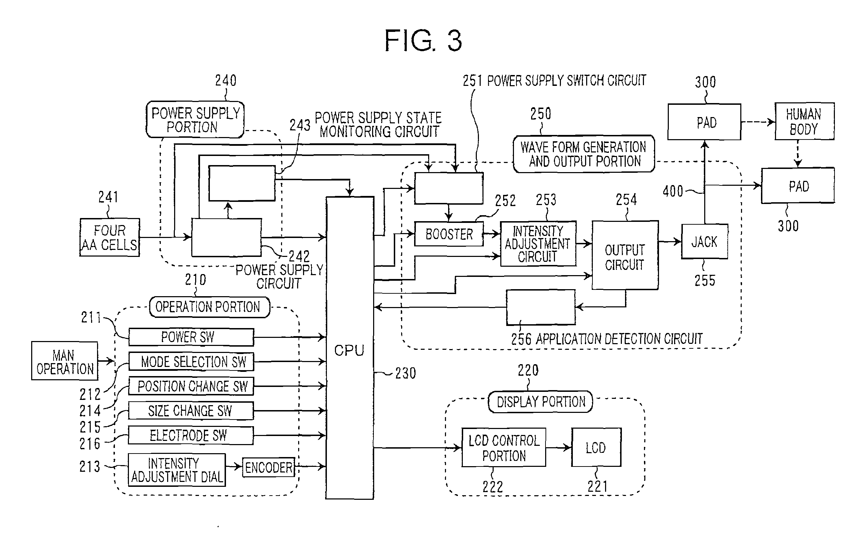

FIG. 3 is a schematic block diagram showing a hardware configuration of the low-frequency electric therapy apparatus. As shown in FIG. 3, the main body 200 roughly includes: an operation portion 210; a display portion 220; a CPU (control portion) 230; a power supply portion 240; and a wave form generation and output portion 250.

As described above, the operation portion 210 includes various kinds of switches and dials. When a user operates a switch or a dial, a corresponding signal is input to the CPU 230. Moreover, an LCD 221, and an LCD control portion 222 controlling the LCD 221 according to a signal from the CPU 230 are provided in the display portion 220.

A power supply circuit 242 and a power supply state monitoring circuit 243 are provided in a power supply portion 240. The low-frequency electric therapy apparatus uses four AA alkaline batteries as a power supply 241. As the voltages of batteries may be greatly changed (about 7 V through 3.5 V) according to the consumption of the batteries, the power supply circuit 242 generates a drive voltage (DC 3.3 V) to be supplied to the CPU 230 by voltage down and stabilization of the voltages of the batteries. A DC output from the power supply circuit 242 is supplied to the CPU 230, and at the same time, is also input to the power supply state monitoring circuit 243. The power supply state monitoring circuit 243 monitors an output voltage from the power supply circuit 242, and stops the CPU 230 (main body 200) when the output voltage becomes lower than 3.3 V.

The wave form generation and output portion 250 is a functional block for increasing an voltage of the power-supply voltage, and for generating and outputting a treating current (treatment wave form), and includes: a power supply switch circuit 251; a booster circuit 252; an intensity adjustment circuit 253; an output circuit 254; a jack 255; an application detection circuit 256; and the like. The power supply switch circuit 251 is a circuit for switching the power-supply voltage supplied to the booster circuit 252, and selects a voltage from the power supply (battery) 241 while treating, and a voltage of 3.3 V from the power supply circuit 242 except for treating. The booster circuit 252 is a circuit boosting a power-supply voltage to a predetermined voltage. The intensity adjustment circuit 253 is a circuit adjusting a voltage boosted in the booster circuit 252 to a voltage corresponding to a set intensity. In the present embodiment, the intensity adjustment may be performed in 21 steps of 0 - 20 levels, using the intensity adjustment dial 213, and an intensity step indicating value set by the dial is input to the intensity adjustment circuit 253 through the CPU 230. The output circuit 254 generates a wave form corresponding to a treatment mode, based on the voltage adjusted in the intensity adjustment circuit 253, and the treatment wave form is output to the electrode 301 of the pad 300 through the jack 255. When operations such as switching of the treatment mode, changing of the energizing area, selection of an electrode, and the like are performed in the operation portion 210, a control signal corresponding to the operation content is input from the CPU 230 to the output circuit 254. The output circuit 254 performs generation of a treatment wave form, switching of a polarity, and switching conducting/non-conducting of each electrode according to the control signal.

The application detection circuit 256 is a circuit which determines whether the pad 300 is correctly applied to the human body by detecting whether an electric current is flowingbetween the pair of pads 300. The above circuit uses a principle that an electric current does not flow when the pad 300 is not correctly applied, because there is no current loop in which an electric current is output from one of the pads 300, passes through the human body, and returns to the other pad 300.

### <Operation at Treating>

The operation method and the treatment operation of a low-frequency electric therapy apparatus 100 will be explained.

A user is able to select a treatment mode by operating the operation portion 210 (mode selection switch 212). A treatment method such as "kneading", "tapping", "pushing", and "rubbing", a treatment portion such as "shoulder", "arm", and "waist", and the like may be specified as a treatment mode. An "automatic" mode in which a treatment method has been programmed beforehand may be also selected. Moreover, a user may also adjust the intensity by operating the dial.

Moreover, with regard to "kneading" and "rubbing", switching between "ordinary mode" and "3D mode" may be switched by operating the mode selection switch 212. The "ordinary mode" is a mode in which the kneading stimulus or the rubbing stimulus is produced by changing the wave form of the treating current with the position and the area of the energizing area keeping fixed, and the bodily sensation similar to that of a conventional low-frequency electric therapy apparatus may be obtained in this mode. The "3D mode" (three-dimensional mode) is a mode in which the kneading stimulus or the rubbing stimulus is produced by changing the position and/or the area of the energizing area while a treating current is output, and a dynamic and three-dimensional bodily sensation which a conventional low-frequency electric therapy apparatus has never provided may be obtained in this mode. The purpose of switching between the ordinary mode and the 3D mode is to improve the convenience of the low-frequency electric therapy apparatus by adequate use of two modes (stimulus) to suit the preferences of the user.

The operation common to that of the ordinary mode and that of the 3D mode will be first explained, and unique operations to each mode will be explained thereafter.

### (Common Operation)

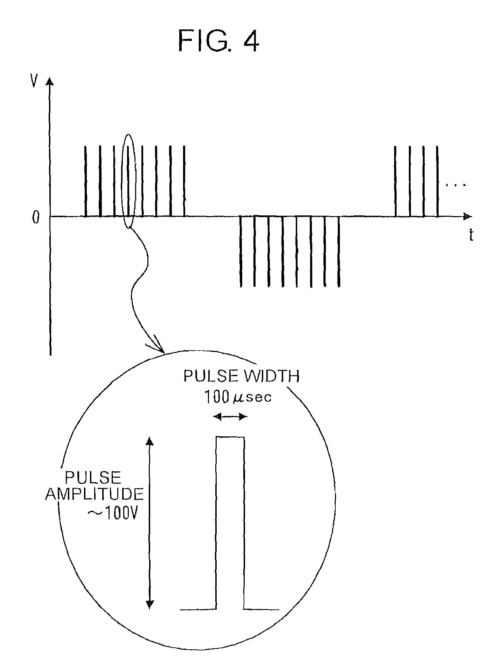

An output circuit 254 generates a treating current of low frequency according to a control signal from the CPU 230. FIG. 4 is a drawing showing one example of treatment wave forms using pulse currents. A pulse with an extremely short width of about 100 microseconds is used as a pulse. The maximum value of the pulse amplitude is about 100 V. A various kinds of stimuli such as "kneading", "tapping", "pushing", and "rubbing" may be produced by changing the wave forms (including polarities, intervals, and arrangements) of the pulses and the like. Moreover, the "intensity" adjustment may be realized by changing the pulse amplitude and the pulse width, and the "speed" adjustment may be realized by changing the occurrence cycle of the pulse group. As a conventional method (refer to, for example, Japanese Patent Application Laid-open Publication No. 2000-14802) may be used for specific wave forms, detailed explanation will be omitted. Here, it is acceptable to use an alternating current, instead of a pulse current, as a treating current.

The treating current is output to the electrode 301 of the pad 300 on the higher potential side (when a plurality of electrodes are in a conducting state, an electric current in one system is distributed to the plurality of conducting electrodes). Then, an electric current flows from the electrode 301 of the pad 300 on the higher potential side to the electrode 301 of the pad 300 on the low potential side through the human body. A muscle receives an electric stimulus by the treating current flowing between the above pads, and repeats contraction and relaxation to obtain a similar treating effect to that of the massaging.

### (Ordinary Mode)

In the ordinary mode, the position and the area of the energizing area are fixed, and are not automatically changed. However, a user may change the position and the area of the energizing area by operating (that is, by manually operating) the position change switch 214 and the size change switch 215 on the operation portion 210.

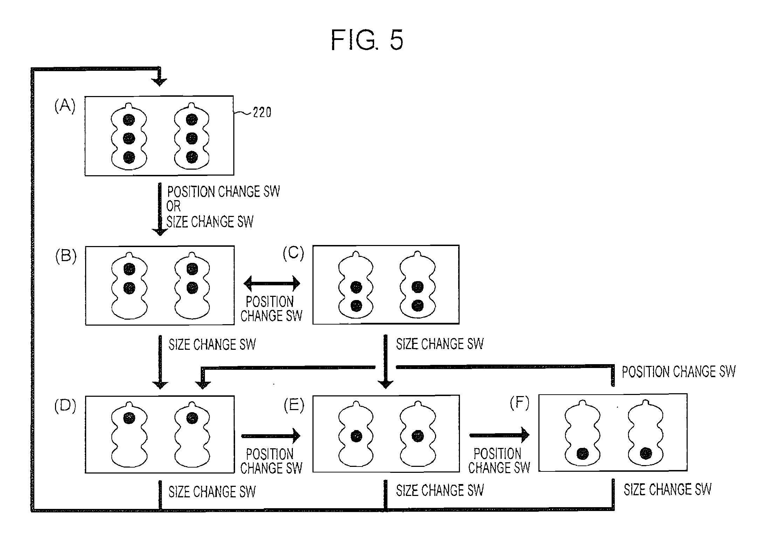

At the starting point (standard state) of the treatment operation, all the six electrodes 301 are in the conducting state and the whole surface of the pad 300 is the energizing area. FIG. 5A shows a display example of the display portion 220 in the standard state. Frames showing external shapes of the pads 300 are drawn on the display portion 220, and marks corresponding to electrodes in a conducting state are in a lighting state. (Hereinafter, for convenience of explanation, three electrodes 301 on the pad 300 are called "upper electrode", "intermediate electrode", and "lower electrode" corresponding to the display on the display portion 220).

Here, when the position change switch 214 (or, the size change switch 215) is pushed once, the output circuit 254 puts the upper and intermediate electrodes of each pad 300 into a conducting state, and the lower electrode of each pad 300 into a cut off (non-conducting) state (refer to FIG. 5B). Thereby, the area in the upper 2/3 portion of the pad 300 becomes an energizing area in which the treating current flows. When the position change switch 214 is further pushed under the above condition, the output circuit 254 puts the upper electrodes of each pad 300 into a cut off state, and the intermediate and the lower electrodes into the conducting state (refer to FIG. 5C). Thereby, the energizing area is moved downwards by one electrode, and the treating current flows only in the lower 2/3 portions of the pad 300. Hereinafter, whenever the position change switch 214 is pushed, the energizing area is moved between the state shown in FIG. 5B and the state shown in FIG. 5C.

Moreover, when the size change switch 215 is pushed under the state shown in FIG. 5B, the output circuit 254 puts only the upper electrodes of each pad 300 into the conducting state, and the intermediate electrode and the lower electrode into the cut off state (refer to FIG. 5D). Thereby, the energizing area becomes further smaller, and the treating current flows only in the area in the upper 1/3 portion of the pad 300. Under the above condition, the energizing area is moved by one electrode in the order, such as the upper electrode → the intermediate electrode → the lower electrode → the upper electrode, ... (refer to FIG. 5D through FIG. 5F) whenever the position change switch 214 is pushed. Moreover, when the size change switch 215 is pushed, all electrodes are put into the conducting state, that is, the state returns to the standard state.

As described above, in the ordinary mode, a user may freely perform switching between wide range treatment and pin point treatment, and adjusting the stimulus position in treatment operation. Accordingly, the operability and the convenience of the low-frequency electric therapy apparatus may be improved.

(3D Mode) In the 3D mode, the CPU 230 automatically changes the position and the area of the energizing area according to a program which has been set beforehand. Bodily sensation that a stimulus position or a force applying angle is changed may be produced by changing the position and the area of the energizing area, and a bodily sensation very similar to those of "kneading", and "rubbing" performed by a person may be given to a user.

Though there are considered a large number of methods (called "changed pattern" or "energizing pattern") for changing an energizing area, it has been found according to the experiments of the present inventors that, when the position or the area of the energizing area, or both of them are changed at a comparatively slow speed of once in about 0.5 seconds through 5 seconds, a bodily sensation very similar to that of "kneading" performed by a person may be obtained. Moreover, it has been found that, when the position or the area of the energizing area or both of them is changed at a comparatively fast speed of once in about 0.1 seconds through 0.5 seconds, a bodily sensation similar to "rubbing" performed by a person may be obtained. Moreover, it has been found that a bodily sensation very similar to that of "kneading" performed by a person may be obtained by alternately making an electrode on end portion and an electrode in the center portion of the pad conductive, or by alternately making all the electrode and a part of the electrodes on the pad conductive. It has been also found that there may be produced a bodily sensation very similar to that of "rubbing" performed by a person when the conducting electrode is moved one by one in the pad. It is preferable to design energizing patterns for each of "kneading" and "rubbing", based on the above experimental results. Hereinafter, specific examples will be given.

### (1) Energizing Pattern for "Kneading"

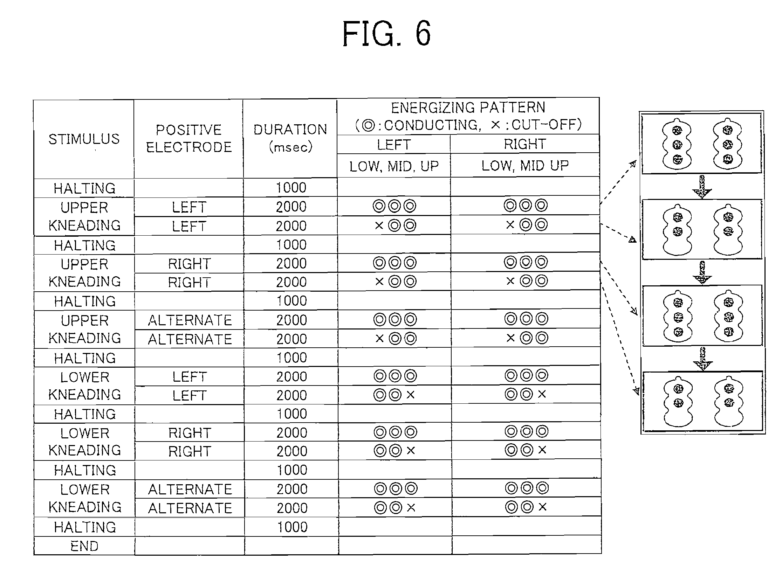

FIG. 6 through FIG. 8 show examples of energizing patterns for "kneading" in the 3D mode.

FIG. 6 shows examples in which symmetrical kneading stimuli are output from the both the right and the left pads under a state in which the energizing patterns for the two pads are kept synchronized. In the table, "halt" shows a state in which the treating current is not output. A treating current with a kneading wave form flows between the right and left pads during an "upper-side kneading" and a "lower-side kneading". "Left" and "right" in the "positive electrode" columns show the higher potential side. "Left" means that a current flows from the left pad to the right pad, and, "right" means that a current flows from the right pad to the left pad. In the case of "alternating", a right and left polarities are switched at a high speed (for example, every 1 msec). Symbols of "⊚ (conducting)" and "× (cut off)" show an electrode state. In the "energizing pattern" column, these symbols are corresponding to, from left to right, the states of the lower electrode, the intermediate electrode, and the upper electrode of the left pad, and similarly, to those of the lower electrode, the intermediate electrode, and the upper electrode of the right pad. Display examples of the display portion 220 are shown at the right side of the table.

When the treatment operation starts, after halting of 1000 msec, a current flows from all the electrodes of the left pad to all the electrodes of the right pad for 2000 msec, and subsequently, the lower electrodes of the both pads are put into a cut off state, in which a current flows from the upper electrode and the intermediate electrode of the left pad to the upper electrode and the intermediate electrode of the right pad during 2000 msec. While there is stimulus all over the pad during the first 2000 msec, the area of the energizing area becomes small, and, at the same time, the center position is shifted upward during the subsequent 2000 msec. Accordingly, a comparatively stronger stimulus is caused in the upper portion of the pad. Thereby, there is caused a bodily sensation that force is slowly applied to the upper portion of the pad. The above operation is corresponding to one time of "upper-side kneading". Three times of "upper-side kneading" are executed placing the halting of 1000 msec between the upper-side kneading.

Subsequently, three times of "lower-side kneadings" are executed. The bodily sensation at this time becomes a bodily sensation that force is slowly applied to the lower side of the pad. Here, as the appearance of changes in the energizing area of each of the pads is displayed on the display portion 220, a user may easily confirm what type of massaging is performed.

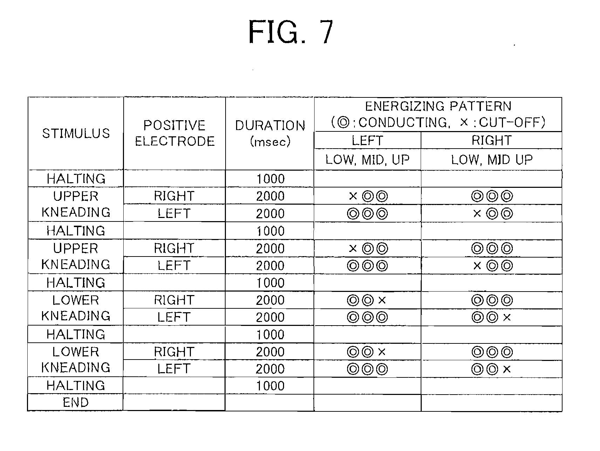

FIG. 7 shows examples in which kneading stimuli which are symmetrical each other are output from the both right and left pads by inverting the energizing patterns oftwopads. In the "upper-side kneading" in this example, a current flows from all the electrodes of the right pad to the upper and the intermediate electrodes of the left pad during the first 2000 msec, and, during the subsequent 2000 msec, a current flows from all the electrodes of the left pad to the upper and the intermediate electrodes of the right pad. When the energizing patterns of the both pads are kept synchronized as shown in FIG. 6, a bodily sensation that right and left treatment portions are kneaded at the same time is obtained, and, when the energizing patterns of the both pads are inverted as shown in FIG. 7, a bodily sensation that right and left treatment portions are alternately kneaded once is obtained. Here, the reason why the pad having a larger area has a positive electrode is that stronger bodily sensation is obtained by the negative electrode.

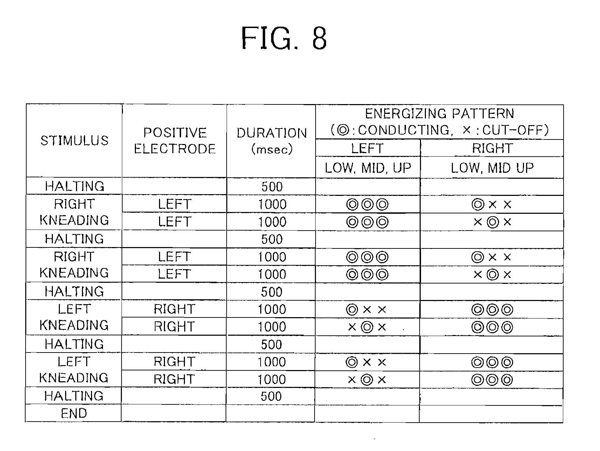

FIG. 8 shows examples in which the area of the energizing area of one pad is fixed at the maximum, and a kneading stimuli is output from the other pad. Moreover, the kneading stimulus is produced by changing only the position of the energizing area in FIG. 8.

When the treatment operation starts, after the halting of 500 msec, a current flows from all the electrodes of the left pad to the lower electrode of the right pad for 1000 msec and a current flows from all electrodes of the left pad to the intermediate electrode of the right pad for the subsequent 1000 msec. This corresponds to one time "right kneading". As the stimuli has characteristics that the smaller the area of the energizing area is, the stronger the stimulus becomes, a weaker static stimulus is output from the left pad, and a dynamic kneading stimulus is output from the right pad. Accordingly, a user experience a bodily sensation as if a person applies the palm of one hand gently onto a treatment portion, and kneading is given to the user by the u hand.

Then, two times of "right kneadings" are executed placing the halting of 500 msec between the right kneading. Thereafter, the right and left kneading changes places, and two times of "left kneading" is performed. In the "left kneading", a current flows from all the electrodes of the right pad to the lower electrode of the left pad for 1000 msec. A current flows from all the electrodes of the right pad to the intermediate electrode of the leftpad for the subsequent 1000 msec. As described above, a bodily sensation that of alternately performed massaging may be given to a user by periodically alternating the pad having the fixed area and the pad producing a stimulus.

### (2) Energizing Pattern of "Rubbing"

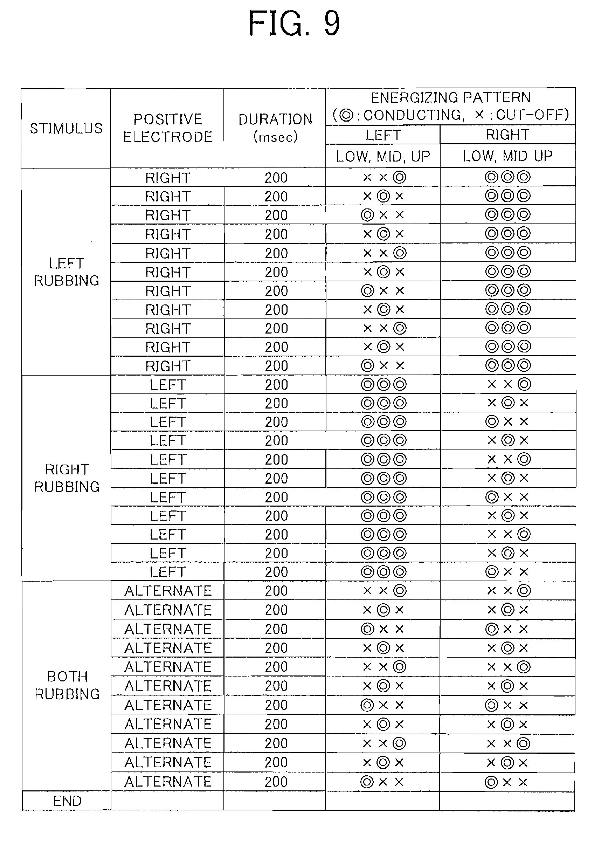

FIG. 9 is a drawing showing one example of the energizing pattern of "rubbing" in the 3D mode.

When the treatment operation starts, "left rubbing", "right rubbing", and "both rubbing" are executed in this order.
In the "left rubbing", the area of the energizing area of the right pad is fixed at the maximum, and a rubbing stimulus is output from the left pad. Specifically, the energizing area (one electrode) of the left pad is moved one by one every 200 msec under a state in which all the electrodes of the right pad are kept in a conducting state. Thereby, the stimulus position in the left pad is reciprocated up and down, and a bodily sensation extremely similar to "rubbing" performed by a person may be produced.

In the subsequent "right rubbing", the right and left kneadings changes places, and, under a state in which all the electrodes of the left pad are kept in a conducting state, the energizing area of the right pad reciprocates and a rubbing stimulus is output to a right treatment portion. Then, in "both rubbing", the energizing areas of the both pads are similarly changed (moved), and a symmetrical rubbing stimulus is output from both pads.

As described above, a bodily sensation that the stimulus position and the force applying angle are changed may be produced and a bodily sensation very similar to "kneading" and "rubbing" performed by a person may be given to a user in the 3D mode. Moreover, as there are two pads, treatment may be simultaneously given to portions mutually apart from each other, such as the right and the left shoulders, the right and the left sides of the waist and the back, the right arm and the left arm, and the right leg and the left leg. Moreover, as the position and the area of the energizing area may be changed by each of the two pads, a bodily sensation that "kneading" and "rubbing" are given to each portion by the both hands maybeproduced. Moreover, since only two pads are needed to be applied, the application is simple. Additionally, there is another advantage that only a simple, and low-cost configuration is required for the above configuration, because there is required, as a current path, only one system between the energizing area of one pad and that of the other pad.

Here, the above-described embodiment only shows one specific example of the present invention. The scope of the present invention is not limited to the above-described embodiment, and many kinds of variations may be realized within a scope of the technological idea of the present invention.

For example, the number of pad electrodes is not limited to three. It is acceptable to use two, or more than three for the numbers. The shape of the electrode and how to divide the electrode are arbitrary. It is acceptable to two-dimensionally change the position and the size of the energizing area by dividing in the transverse direction as well as in the longitudinal direction.

## Claims

1. A low-frequency electric therapy apparatus, comprising:
a pair of pads each of which is capable of changing the position and the area of an energizing area; and
an output portion which applies a treating current between the energizing area of one pad and that of the other pad, wherein
said output portion produces a kneading stimulus or a rubbing stimulus by changing the position and/or the area of the energizing area of at least one pad while outputting the treating current.

2. A low-frequency electric therapy apparatus as claimed in claim 1, wherein
in the case of kneading stimulus, said output portion changes said energizing area at a speed of once in 0.5 seconds through 5 seconds.

3. A low-frequency electric therapy apparatus as claimed in claim 1 or 2,
in the case of rubbing stimulus, said output portion changes said energizing area at a speed of once in 0.1 seconds through 0.5 seconds.

4. A low-frequency electric therapy apparatus as claimed in any one of claims 1 through 3, wherein
said pad has a plurality of electrodes which may be individually switched between conducting and non-conducting states, and
said output portion changes the position and the area of said energizing area by changing a conducting electrode.

5. A low-frequency electric therapy apparatus as claimed in claim 4, wherein
said pad has an electrode on an end portion and an electrode in a center portion, and
said output portion produces a kneading stimulus by alternately making the electrode on said end portion and the electrode in said center portion conductive.

6. A low-frequency electric therapy apparatus as claimed in claim 4, wherein
said output portion produces a kneading stimulus by alternately making all the electrode and a part of the electrodes on the pad conductive.

7. A low-frequency electric therapy apparatus as claimed in any one of claims 4 through 6, wherein
said output portion produces a rubbing stimulus by moving a conducting electrode one by one in the pad.

8. A low-frequency electric therapy apparatus claimed in any one of claims 1 through 7, wherein
said output portion outputs symmetrical kneading stimulus or rubbing stimulus from the both pads by making the change patterns of the energizing areas for the two pads in synchronized with each other, or by mutually inverting the change patterns.

9. A low-frequency electric therapy apparatus as claimed in any one of claims 1 through 7, wherein
said output portion sets the area of the energizing area of one pad at a maximum value, and a kneading stimulus or a rubbing stimulus is output from the other pad.

10. A low-frequency electric therapy apparatus as claimed in claim 9, wherein
said output portion periodically alternate the pad having the fixed area of said energizing area and the pad outputting a kneading stimulus or a rubbing stimulus.

11. A low-frequency electric therapy apparatus as claimed in any one of claims 1 through 10, wherein
said output portion may switch between
a first mode in which a kneading stimulus or a rubbing stimulus is produced by changing the position and/or the area of an energizing area of at least one pad while outputting a treating current; and
a second mode in which a kneading stimulus or a rubbing stimulus is produced by changing the wave form of a treating current under a state in which the position and the area of the energizing area are fixed.

12. A low-frequency electric therapy apparatus as claimed in any one of claims 1 through 11, further comprising a display portion showing energizing areas in each pad.
